# EUROPEAN PATENT APPLICATION

(11) **EP 3 081 922 A1**
(43) Date of publication of application: **19.10.2016**
(21) Application number: 16163780.6
(22) Date of filing: 05.04.2016
(51) Int. Cl.: G01N 21/3504, G01J 3/28, G01J 3/42, G01N 21/3518, G01N 21/359, G01N 21/39

(54) **GAS CONCENTRATION ANALYZER AND GAS CONCENTRATION ANALYSIS METHOD**

(30) Priority: 14.04.2015 JP 2015082361
(71) Applicant: Yokogawa Electric Corporation, Tokyo 180-8750 (JP)
(72) Inventor: Ooyama, Masaya, Tokyo, 180-8750 (JP); Mitsumoto, Yasuhiko, Tokyo, 180-8750 (JP); Fujimura, Naoyuki, Tokyo, 180-8750 (JP); Murata, Akihiro, Tokyo, 180-8750 (JP)
(74) Representative: Henkel, Breuer & Partner

(57) **Abstract**

A gas concentration analyzer includes: a light emitting module (10) configured to emit a laser light with which an ethylene gas that is a measurement target gas (X) is irradiated; a light receiving module (20) configured to receive the laser light that has passed through the measurement target gas to output a light receiving signal; and a calculation controller (22) configured to calculate an absorption spectrum of the laser light using the light receiving signal, and to calculate amounts of methane and amounts of ethane in the ethylene gas that is the measurement target gas based on the absorption spectrum, wherein the laser light with which the measurement target gas is irradiated is included in a wavelength region with a wavelength of 3.42 µm or more and 3.71 µm or less.

## Description

### BACKGROUND OF THE INVENTION

### Field of the Invention

The present invention relates to a gas concentration analyzer and a gas concentration analysis method.

Priority is claimed on Japanese Patent Application No. 2015-082361, filed on April 14, 2015, the content of which is incorporated herein by reference.

### Description of Related Art

All patents, patent applications, patent publications, scientific articles, and the like, which will hereinafter be cited or identified in the present application, will hereby be incorporated by reference in their entirety in order to describe more fully the state of the art to which the present invention pertains.

Ethylene (C₂H₄) is produced in production facilities referred to as ethylene plants by mixing hydrocarbons, mainly naphtha, with water vapor, thermally decomposing the mixture at a high temperature of approximately 800°C to 900°C, and distilling and separating the pyrolysis gas. Since methane (CH₄) and ethane (C₂H₆) are included as impurities in the pyrolysis gas, a step of removing methane and ethane from the pyrolysis gas is carried out during a purification step. However, since it is difficult to completely remove methane and ethane from the pyrolysis gas, trace amounts of methane and ethane are included in ethylene as the final product. For this reason, the trace amounts of methane and ethane which are included in the produced ethylene are measured in the ethylene plant.

In the related art, the trace amounts of methane and ethane which are included in ethylene are measured using gas chromatography. It is possible to measure the trace amounts of methane and ethane which are included in ethylene with high precision using gas chromatography. However, gas chromatography has disadvantages in that the measurement takes a long time and the cost is high since it is necessary to use a carrier gas (for example, helium, hydrogen, or the like) in order to move the measurement target gas.

Japanese Unexamined Patent Application, First Publication No. 2009-115654 (referred to below as patent literature 1) does not relate to the measurement of trace amounts of methane and ethane which are included in ethylene; however, patent literature 1 discloses calculating the sum of the concentrations of chemical types which absorb light in a wavelength band in a common absorption region from among one or a plurality of chemical types which are included in the measurement target gas based on the absorbance of light with which the measurement target gas which includes hydrocarbons is irradiated. In addition, "Laser Gas Analyzer TDLS 200 and Application to Industrial Processes", Yokogawa technical report, Vol. 53, No. 2, 2010 (referred to below as non-patent literature 1) discloses a laser gas analyzer which irradiates the measurement target gas with the laser light and measures components which are included in the measurement target gas, the concentrations thereof, or the like based on the absorption spectrum of laser light which passes through the measurement target gas.

### SUMMARY OF THE INVENTION

Here, in the technique which is disclosed in patent literature 1, it is possible to measure the concentration of hydrocarbons which are included in the measurement target gas in real time with high precision even when the measurement target gas is a gas where the concentration and composition of the hydrocarbons change such as an engine exhaust gas or the like. However, since the technique which is disclosed in patent literature 1 is for detecting the sum of the concentrations of chemical types (for example, the sum of the alkane and alkene concentrations), it is not possible to individually measure the trace amounts of methane and ethane which are included in ethylene.

In the laser gas analyzer which is disclosed in non-patent literature 1, it is considered that it is possible to individually measure the trace amounts of methane and ethane which are included in ethylene. However, for the laser gas analyzer which is disclosed in non-patent literature 1, the range in which it is possible to tune the wavelength is limited to a certain extent. For this reason, to individually measure the trace amounts of methane and ethane which are included in ethylene, a light source and light receiving elements for measuring methane are necessary and a light source and light receiving elements for measuring ethane are necessary and there is a possibility that the cost will increase. In order to measure the trace amounts of methane and ethane which are included in ethylene without increasing the cost, for example, it is necessary to examine and select an appropriate wavelength region in which the absorbance of ethylene is as small as possible and the absorbance of both methane and ethane is great.

The present invention provides a gas concentration analyzer which is capable of measuring trace amounts of methane and ethane which are included in ethylene with high precision.

A gas concentration analyzer includes: a light emitting module configured to emit a laser light with which an ethylene gas that is a measurement target gas is irradiated; a light receiving module configured to receive the laser light that has passed through the measurement target gas to output a light receiving signal; and a calculation controller configured to calculate an absorption spectrum of the laser light using the light receiving signal, and to calculate amounts of methane and amounts of ethane in the ethylene gas that is the measurement target gas based on the absorption spectrum, wherein the laser light with which the measurement target gas is irradiated is included in a wavelength region with a wavelength of 3.42 µm or more and 3.71 µm or less.

The laser light with which the measurement target gas is irradiated may be included in a wavelength region with a wavelength of 3.42 µm or more and 3.50 µm or less.

The laser light with which the measurement target gas is irradiated may be included in a wavelength region with a wavelength of 3.439 µm or more and 3.442 µm or less or 3.451 µm or more and 3.455 µm or less.

The laser light with which the measurement target gas is irradiated may have a spectrum line width which is equivalent to or less than a spectrum line width of an absorption peak of methane or ethane.

The gas concentration analyzer may include a tunable wavelength light source configured to be capable of changing the wavelength of the laser light with which the measurement target gas is irradiated within the wavelength region.

The gas concentration analyzer may include only one tunable wavelength light source. The methane and the ethane which are included in the ethylene gas that is the measurement target gas can be calculated simultaneously using the laser light emitted from the tunable wavelength light source.

The tunable wavelength light source may be a quantum cascade laser (QCL) or an interband cascade laser (ICL).

A gas concentration analysis method includes: emitting a laser light with which an ethylene gas that is a measurement target gas is irradiated; receiving the laser light that has passed through the measurement target gas to output a light receiving signal; calculating an absorption spectrum of the laser light using the light receiving signal; and calculating amounts of methane and amounts of ethane in the ethylene gas that is the measurement target gas based on the absorption spectrum, wherein the laser light with which the measurement target gas is irradiated is included in a wavelength region with a wavelength of 3.42 µm or more and 3.71 µm or less.

The laser light with which the measurement target gas is irradiated may be included in a wavelength region with a wavelength of 3.42 µm or more and 3.50 µm or less.

The laser light with which the measurement target gas is irradiated may be included in a wavelength region with a wavelength of 3.439 µm or more and 3.442 µm or less or 3.451 µm or more and 3.455 µm or less.

The laser light with which the measurement target gas is irradiated may be a laser light having a spectrum line width which is equivalent to or less than a spectrum line width of an absorption peak of methane or ethane.

The wavelength of the light with which the measurement target gas is irradiated may be tuned within the wavelength region by a tunable wavelength light source.

The methane and the ethane, which are included in the ethylene, may be calculated simultaneously using the laser light which is emitted from the tunable wavelength light source.

The tunable wavelength light source may be a quantum cascade laser (QCL) or an interband cascade laser (ICL).

According to an aspect of the present invention, a gas concentration analyzer is a gas concentration analyzer (1) which irradiates a measurement target gas (X) with light (L) and measures a concentration of the measurement target gas based on the absorbance of the light, in which the measurement target gas is ethylene in which trace amounts of methane and ethane are included and the light with which the measurement target gas is irradiated is light which is included in a wavelength region (R1) with a wavelength of 3.42 µm or more and 3.71 µm or less.

In addition, according to an aspect of the present invention, in the gas concentration analyzer, the light with which the measurement target gas is irradiated is light which is included in a wavelength region (R2) with a wavelength of 3.42 µm or more and 3.50 µm or less.

In addition, according to an aspect of the present invention, in the gas concentration analyzer, the light with which the measurement target gas is irradiated is light which is included in wavelength regions (R3 and R4) with a wavelength of 3.439 µm or more and 3.442 µm or less or 3.451 µm or more and 3.455 µm or less.

In addition, according to an aspect of the present invention, in the gas concentration analyzer, the light with which the measurement target gas is irradiated is laser light with a spectrum line width which is equivalent to or less than a spectrum line width of an absorption peak of methane or ethane.

In addition, according to an aspect of the present invention, the gas concentration analyzer includes a tunable wavelength light source which is capable of changing the wavelength of the light with which the measurement target gas is irradiated within the wavelength region.

In addition, according to an aspect of the present invention, the gas concentration analyzer includes only one tunable wavelength light source and the methane and the ethane which are included in the ethylene are measured simultaneously using the laser light which is emitted from the tunable wavelength light source.

According to the aspects of the present invention, since the concentration of a measurement target gas X is measured based on the absorbance of the irradiated light by irradiating the measurement target gas X (ethylene in which trace amounts of methane and ethane are included) with light which is included in a wavelength region (a wavelength region R1) with a wavelength of 3.42 µm or more and 3.71 µm or less, there is an effect in which it is possible to measure the trace amounts of methane and ethane which are included in ethylene with high precision.

### BRIEF DESCRIPTION OF THE DRAWINGS

The above features and advantages of the present invention will be more apparent from the following description of certain preferred embodiments taken in conjunction with the accompanying drawings, in which:
Fig. 1 is a graph showing an absorption spectrum at 7.3 µm to 8.6 µm which is a candidate wavelength region which is suitable for the detection of trace amounts of methane;
Fig. 2 is a graph showing an absorption spectrum of a 6.5 µm band which is one candidate wavelength region which is suitable for the detection of trace amounts of ethane;
Fig. 3 is a graph showing an absorption spectrum of a 3.4 µm band which is another candidate wavelength region which is suitable for the detection of trace amounts of ethane;
Fig. 4 is a graph showing an absorption spectrum of ethylene in the 3.4 µm band which was obtained by request from an external organization;
Fig. 5A is an enlarged view of the absorption spectrum of ethylene in the 3.4 µm band which was obtained by request from an external organization;
Fig. 5B is an enlarged view of the absorption spectrum of ethylene in the 3.4 µm band which was obtained by request from an external organization;
Fig. 6 is an enlarged view of the absorption spectrum of ethylene in the 3.4 µm band which was obtained by request from an external organization;
Fig. 7 is an enlarged view of the horizontal axis in Fig. 6;
Fig. 8 is a block diagram showing the main configuration of a gas concentration analyzer according to one embodiment of the present invention; and
Fig. 9 is a block diagram showing an electrical configuration of a gas concentration analyzer according to one embodiment of the present invention.

### DETAILED DESCRIPTION OF THE INVENTION

The present invention will be now described herein with reference to illustrative preferred embodiments. Those skilled in the art will recognize that many alternative preferred embodiments can be accomplished using the teaching of the present invention and that the present invention is not limited to the preferred embodiments illustrated herein for explanatory purposes.

Detailed description will be given below of a gas concentration analyzer according to an embodiment of the present invention with reference to drawings. The gas concentration analyzer according to the embodiment of the present invention is capable of individually measuring trace amounts of methane and ethane which are included in ethylene. In the gas concentration analyzer, in order to measure trace amounts of methane and ethane which are included in ethylene without increasing costs, for example, it is extremely important to examine and select an appropriate wavelength region in which the absorbance of ethylene is as small as possible and the absorbance of both methane and ethane is great. For this reason, description will be given below of the wavelength of light which is used in the gas concentration analyzer before description is given of the apparatus configuration of the gas concentration analyzer.

### (Wavelength of Light Used in Gas Concentration Analyzing Apparatus)

The inventor of the present application examined the absorbance of ethylene, methane, and ethane in detail using data which was obtained from an existing database (a database in which data indicating the absorption spectrums of various types of gas is stored). Data indicating the absorbance of ethylene, methane, and ethane with a high purity (a purity close to 100% (for example, a purity of approximately 99.5%)) was obtained from the existing database. According to the data, the wavelength region in which the absorbance of methane is greater than the absorbance of ethylene was the wavelength region of 7.3 µm to 8.6 µm. In addition, the wavelength region in which the absorbance of ethane is greater than the absorbance of ethylene was the 3.4 µm band or the 6.5 µm band. Examples of candidates of wavelength regions (candidate wavelength regions) which are suitable for the measurement of trace amounts of methane and ethane which are included in ethylene include these wavelength regions.

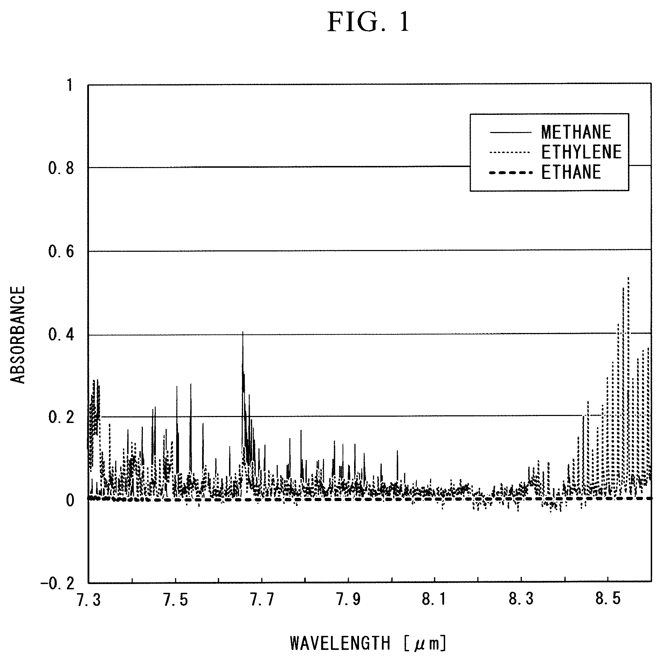

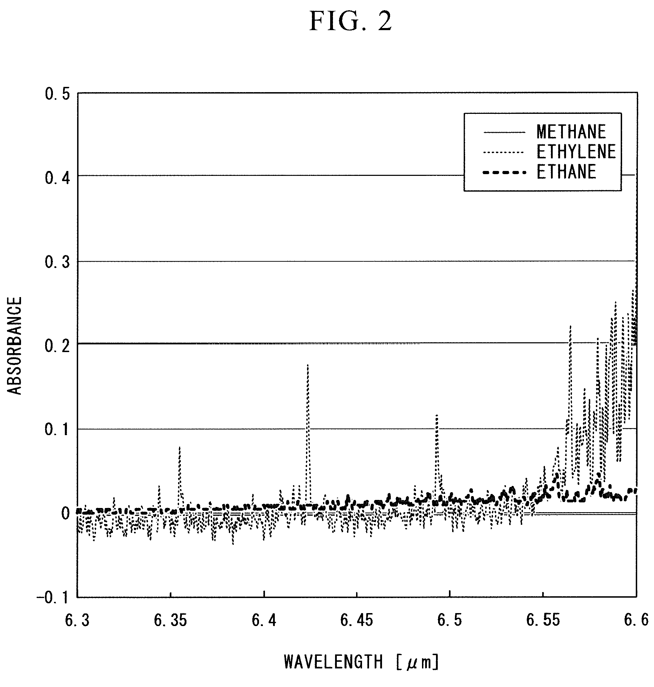

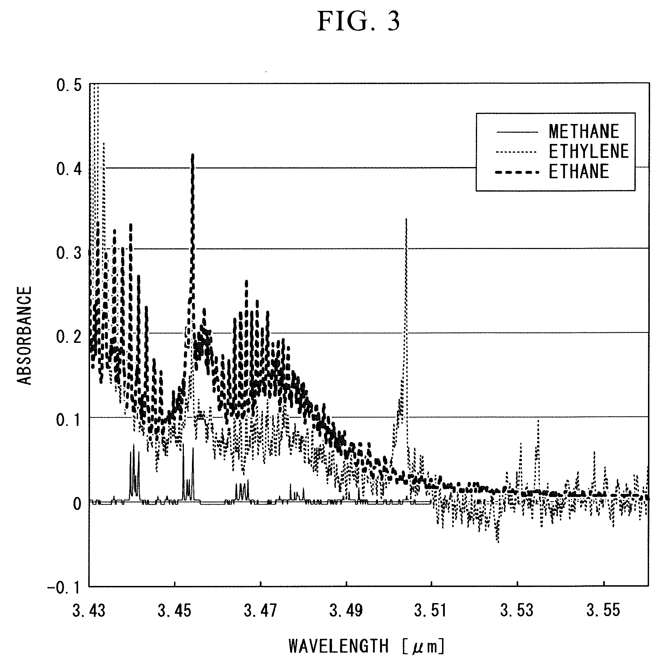

Fig. 1 to Fig. 3 are graphs showing an absorption spectrum in a candidate wavelength region which is suitable for the measurement of trace amounts of methane and ethane which are included in ethylene. Fig. 1 is a graph showing an absorption spectrum at 7.3 µm to 8.6 µm which is a candidate wavelength region which is suitable for the detection of trace amounts of methane. Fig. 2 is a graph showing an absorption spectrum of a 6.5 µm band which is one candidate wavelength region which is suitable for the detection of trace amounts of ethane. Fig. 3 is a graph showing an absorption spectrum of a 3.4 µm band which is another candidate wavelength region which is suitable for the detection of trace amounts of ethane.

Here, the absorption spectrum shown in Fig. 1 to Fig. 3 is produced by processing data which is obtained from the existing database described above. In detail, the absorption spectrum is produced by reducing the vertical axis of the data of methane and ethane with high purity (purity close to 100%) which is obtained from the existing database according to the concentrations thereof and overlapping the results with the data of ethylene with high purity (purity close to 100%). It is possible to say that the absorption spectrum which is produced in this manner will be the same as the absorption spectrum of a measurement target gas where trace amounts of ethane (for example, approximately 500 ppm) and trace amounts of methane (for example, approximately 1500 ppm) are included in ethylene of which the purity is approximately 100%.

Referring to Fig. 1, it is understood that there is a wavelength where the absorbance of methane is greater than the absorbance of ethylene and the shape of the absorption spectrum of methane has a characteristic shape (for example, a shape which has a plurality of peaks). For this reason, it is considered that it is possible to measure trace amounts of methane which are included in ethylene when using light in the wavelength region (7.3 µm to 8.6 µm) shown in Fig. 1. In addition, referring to Fig. 2, it is understood that there is a wavelength where the absorbance of ethane is small compared to the absorbance of methane shown in Fig. 1 but the absorbance of ethane is greater than the absorbance of ethylene. For this reason, it is considered that it is possible to measure trace amounts of ethane which are included in ethylene when using light in the wavelength region (the 6.5 µm band) shown in Fig. 2.

From the above, it is considered that it is possible to measure trace amounts of methane and ethane which are included in ethylene when using light in the wavelength region (7.3 µm to 8.6 µm) shown in Fig. 1 and light in the wavelength region (the 6.5 µm band) shown in Fig. 2. However, in a case of using these wavelength regions, since a light source which emits light in each of the wavelength regions is necessary, it is considered that there is a possibility that the cost will be increased.

Referring to Fig. 3, it is understood that there is a wavelength where the shape of the absorption spectrum of methane is a characteristic shape (for example, a shape which has a plurality of peaks), the absorbance of ethane is greater than the absorbance of ethylene, and the shape of the absorption spectrum of ethane is a characteristic shape. For this reason, when it is possible to use light in the wavelength region (the 3.4 µm band) shown in Fig. 3, it is also considered that there is a possibility that it will be possible to measure trace amounts of methane and ethane which are included in ethylene without using a plurality of light sources (without increasing the cost). However, referring to Fig. 3, a person skilled in the related art would naturally consider that it is not possible to use the wavelength region shown in Fig. 3 since the absorbance of methane is much smaller than the absorbance of ethylene.

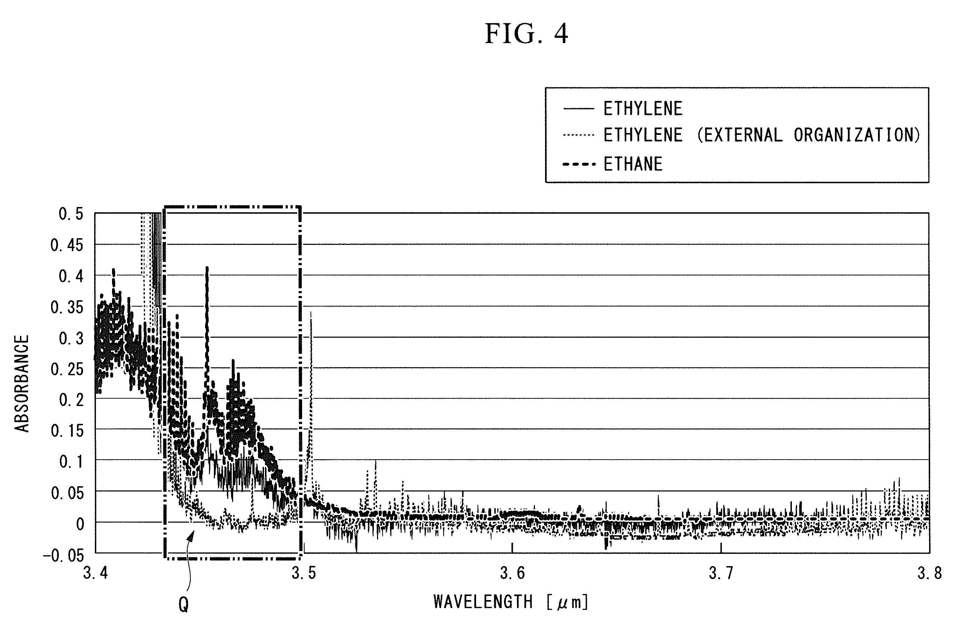

Here, the inventor of the present application made a request to an external organization for the measurements of the absorption spectrum of ethylene with extremely high purity (ethylene with purity of 99.999%) in the 3.4 µm band and examined these measurement results. Fig. 4 is a graph showing the absorption spectrum of ethylene in the 3.4 µm band which was obtained by request from an external organization. In Fig. 4, the waveform shown by the symbol Q is the absorption spectrum of ethylene which was obtained by request from the external organization. Here, in Fig. 4, for comparison, the absorption spectrum of ethylene and the absorption spectrum of ethane shown in Fig. 3 are shown overlapping.

Referring to Fig. 4, it is understood that the absorption spectrum of ethylene which is obtained by request from the external organization is different from the absorption spectrum of ethylene shown in Fig. 3 and that the absorbance in the 3.4 µm band (the wavelength region which is surrounded by the broken line in the drawing) is small as a whole. In addition, referring to Fig. 4, it is also understood that there is a wavelength where the absorbance of ethylene is substantially zero in the absorption spectrum which was obtained by request from the external organization. Due to this, in the 3.4 µm band, it is understood that there is a possibility that there is a wavelength where the absorbance of methane is greater than the absorbance of ethylene and the absorbance of ethane is greater than the absorbance of ethylene.

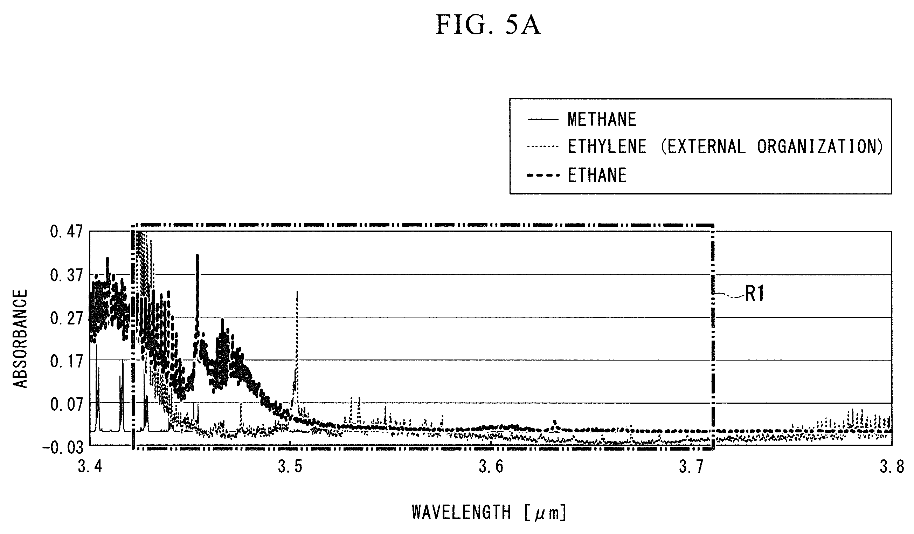

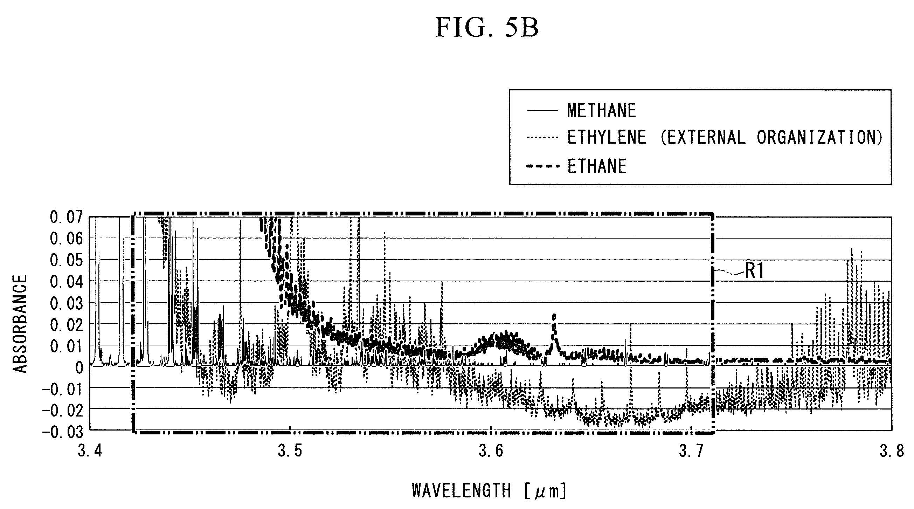

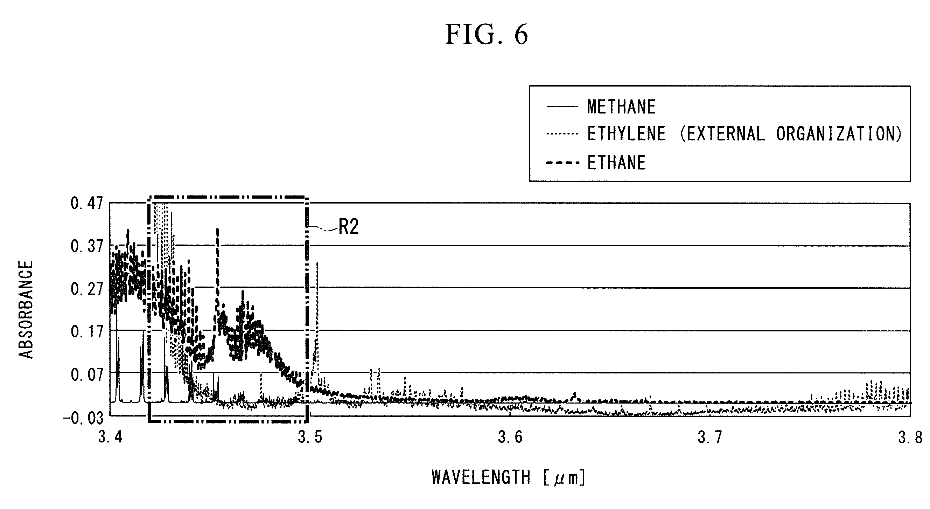

Here, the inventor of the present application performed an operation of specifically selecting a wavelength region which is suitable for measuring trace amounts of methane and ethane which are included in ethylene. Figs. 5A and 5B, and Fig. 6 are enlarged views of the absorption spectrum of ethylene in the 3.4 µm band which was obtained by request from an external organization. Here, Fig. 5B is an enlarged view of the vertical axis in Fig. 5A and Fig. 6 is the same view as Fig. 5A. In addition, in Figs. 5A and 5B, and Fig. 6, the absorption spectrum of methane and ethane shown in Fig. 3 are shown overlapping.

Referring to Figs. 5A and 5B, examples of a wavelength region in which it is possible to measure trace amounts of methane and ethane which are included in ethylene (basically, a region including a wavelength where the absorbance of methane and ethane is greater than the absorbance of ethylene and the shape of the absorption spectrum of methane and ethane is a characteristic shape) include 3.42 µm to 3.71 µm (the wavelength region R1 in Figs. 5A and 5B). In addition, referring to Fig. 6, examples of wavelength regions in which the absorbance of ethane is great, the shape of the absorption spectrum of methane is a characteristic shape and in which it is possible to easily measure methane and ethane in the wavelength region R1 shown in Figs. 5A and 5B include 3.42 µm to 3.50 µm (the wavelength region R2 in Fig. 6).

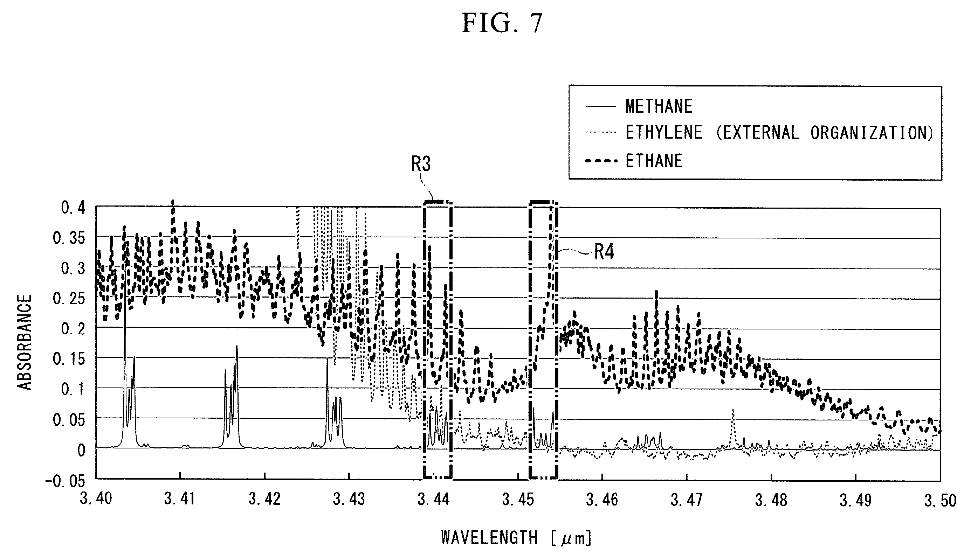

Fig. 7 is an enlarged view of the horizontal axis in Fig. 6. As shown in Fig. 7, examples of a wavelength region which is suitable for measuring trace amounts of methane and ethane which are included in ethylene in the wavelength region R2 shown in Fig. 6 include 3.439 µm to 3.442 µm (the wavelength region R3 in Fig. 7) and 3.451 µm to 3.455 µm (the wavelength region R4 in Fig. 7). In the wavelength regions R3 and R4, not only is the absorbance of ethane greater than the absorbance of ethylene but the absorbance of methane is also great and the absorbance of methane is approximately the same as the absorbance of ethylene (or the absorbance of methane is greater than the absorbance of ethylene). In addition, the shape of the absorption spectrum of methane and ethane is a characteristic shape.

In this manner, the inventors of the present application discovered 3.42 µm to 3.71 µm (the wavelength region R1 in Figs. 5A and 5B) as a wavelength region in which it is possible to measure trace amounts of methane and ethane which are included in ethylene. In addition, the inventors of the present application discovered 3.42 µm to 3.50 µm (the wavelength region R2 in Fig. 6) as a wavelength region in which is it is possible to easily measure ethane in the wavelength region R1 described above. Furthermore, the inventor of the present application discovered 3.439 µm to 3.442 µm (the wavelength region R3 in Fig. 7) and 3.451 µm to 3.455 µm (the wavelength region R4 in Fig. 7) as wavelength regions which are suitable for measuring trace amounts of methane and ethane which are included in ethylene in the wavelength region R3 described above.

### (Gas Concentration Analyzer)

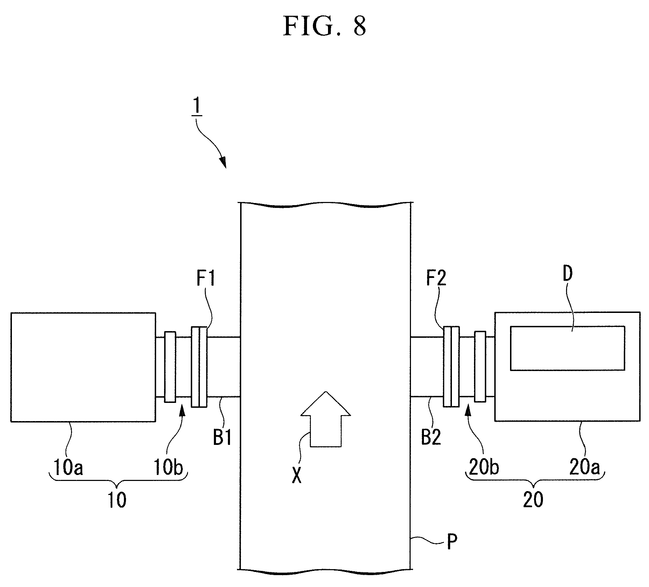

Fig. 8 is a block diagram showing the main configuration of a gas concentration analyzer according to one embodiment of the present invention. As shown in Fig. 8, a gas concentration analyzer 1 of the present embodiment includes a light emitter 10 and a light receiver 20 which are installed opposing each other with a flue (a pipeline P in which the measurement target gas X is guided) interposed therebetween, and measures components which are included in the measurement target gas X, the concentrations thereof, and the like based on the absorption spectrum of the laser light which passes through the measurement target gas by irradiating the measurement target gas X which flows in the pipeline P with laser light. The gas concentration analyzer 1 is called a laser gas analyzer. Here, the measurement target gas X which flows in the pipeline P is ethylene in which trace amounts of methane and ethane are included.

The light emitter 10 is attached to a fixed flange F1 of a branch pipeline B1 which is formed on a side wall of the pipeline P and emits laser light for irradiating the measurement target gas X which flows in the pipeline P. The laser light is laser light which has a wavelength within the wavelength region R1 (3.42 µm to 3.71 µm) in Figs. 5A and 5B, preferably laser light which has a wavelength within the wavelength region R2 (3.42 µm to 3.50 µm) in Fig. 6, and more preferably laser light which has a wavelength within the wavelength region R3 (3.439 µm to 3.442 µm) or the wavelength region R4 (3.451 µm to 3.455 µm) in Fig. 7.

The line width (the spectrum line width) of laser light which is emitted from the light emitter 10 is a spectrum line width which is equivalent to or less than the minimum spectrum line width of an absorption peak of methane or ethane in the wavelength regions R1 to R4. In addition, it is possible to tune the wavelength of the laser light which is emitted from the light emitter 10 only in a predetermined wavelength range within the wavelength regions R1 to R4 described above. Here, the laser light which is emitted from the light emitter 10 is guided to the pipeline P via the inside of the branch pipeline B1.

The light emitter 10 is formed of a light emitter main body 10a and an optical axis adjusting mechanism 10b. The light emitter main body 10a accommodates light emitting elements (a tunable wavelength light source, not shown in the drawing) such as a semiconductor laser where the wavelength of the emitted light is tunable, optical elements (which is not shown in the drawing) such as a collimating lens, and the like, and emits laser light with which the measurement target gas X which flows in the pipeline P is irradiated. The optical axis adjusting mechanism 10b is a mechanism for adjusting the optical axis of the light emitter 10.

The light receiver 20 is attached to a fixed flange F2 of a branch pipeline B2 (a branch pipeline which is formed so as to be arranged on the same one straight line as the branch pipeline B1) which is formed on the side wall of the pipeline P, obtains an absorption spectrum by receiving the laser light which passes through the measurement target gas X which flows in the pipeline P and measures components which are included in the measurement target gas X, the concentrations thereof, and the like based on the absorption spectrum. In detail, the concentrations of trace amounts of methane and ethane which are included in ethylene as the measurement target gas X are measured. Here, the laser light which passes through the measurement target gas X is guided to the light receiver 20 via the inside of the branch pipeline B2.

The light receiver 20 is formed of a light receiver main body 20a and an optical axis adjusting mechanism 20b. The light receiver main body 20a accommodates optical elements (which are not shown in the drawing) such as a condensing lens, light receiving elements (which are not shown in the drawing) such as photodiode, and the like and receives laser light which passes through the measurement target gas X, and measures components which are included in the measurement target gas X, the concentrations thereof, and the like. In addition, display section D which displays the measurement results and the like of the measurement target gas X is provided in the light receiver main body 20a. The optical axis adjusting mechanism 20b is a mechanism for adjusting the optical axis of the light receiver 20.

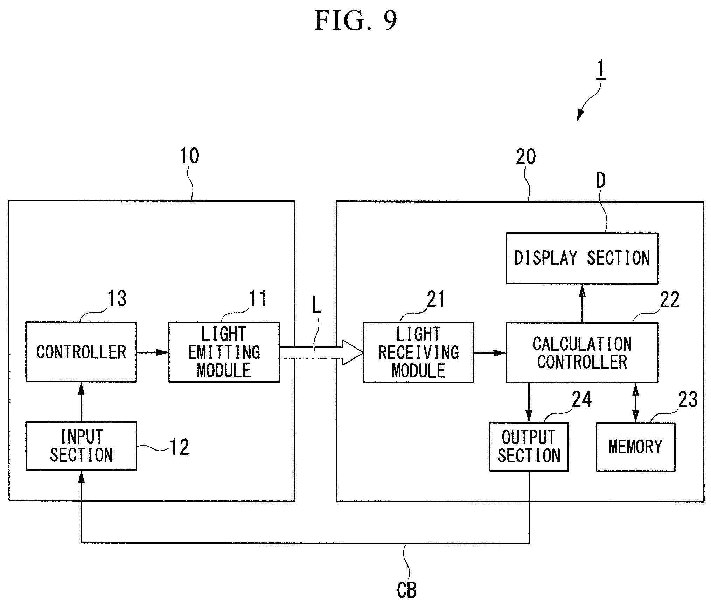

Fig. 9 is a block diagram showing an electrical configuration of a gas concentration analyzer according to one embodiment of the present invention. As shown in Fig. 9, the light emitter 10 of the gas concentration analyzer 1 includes with a light emitting module 11, an input section 12, and a controller 13. In addition, the light receiver 20 of the gas concentration analyzer 1 includes a light receiving module 21, a calculation controller 22, a memory 23, and an output section 24 in addition to the display section D described above.

The light emitting module 11 includes one light emitting element (tunable wavelength light source, not shown in the drawing) such as a semiconductor laser where the wavelength is tunable and emits laser light L with which the measurement target gas X which flows in the pipeline P is irradiated under the control of the controller 13. In detail, the light emitting module 11 emits laser light which has a wavelength within the wavelength regions R1 to R4 shown in Fig. 5A to Fig. 7 with a line width (spectrum line width) equivalent to or less than the minimum spectrum line width (approximately 0.2 nm) of the absorption peak of methane or ethane in the wavelength regions R1 to R4, for example, approximately 0.2 nm. It is possible to use, for example, a quantum cascade laser (QCL) or an interband cascade laser (ICL) as the semiconductor laser described above. In a case of using a QCL, it is possible to emit laser light with a spectrum line width of approximately 0.001 nm.

It is possible to tune the wavelength of the laser light which is emitted from the light emitting module 11 only in a predetermined wavelength range within the wavelength regions R1 to R4 described above. The input section 12 is connected to the output section 24 which is provided in the light receiver 20 via a cable CB and inputs a transmittance signal (a signal which indicates the transmittance of the laser light L) which is output from the output section 24 and output to the controller 13.

The controller 13 emits the laser light L with which the measurement target gas X is irradiated by controlling the light emitting module 11. In detail, the controller 13 controls the light emitting module 11 such that the strength of the laser light L is constant based on the detection results of the current which flows in the light emitting element which is provided in the light emitting module 11 or the detection results of the laser light L which is emitted from the light emitting module 11. In addition, the controller 13 tunes the wavelength of the laser light L by controlling the light emitting module 11 and sweeps a predetermined wavelength range within the wavelength regions R1 to R4 described above. It is possible to form the controller 13 of a CPU, FPGA, or the like.

The light receiving module 21 includes a light receiving element such as a photodiode and outputs a light receiving signal which is obtained by receiving the laser light L to the calculation controller 22. The calculation controller 22 calculates the absorption spectrum of the laser light L using the light receiving signal from the light receiving module 21 and measures components which are included in the measurement target gas X and the concentrations thereof, the transmittance of the laser light L, the temperature of the measurement target gas X, and the like based on this absorption spectrum.

The calculation controller 22 measures the concentration of the components which are included in the measurement target gas X using, for example, the 2f method or a spectrum area method. Here, the 2f method described above is a measuring method in which the measurement target gas X is irradiated with the laser light L which is tuned in the frequency f and measured using a spectrum of twice the frequency components (2f) included in the light receiving signal. In addition, the spectrum area method is a measuring method in which an area of one absorption line of the absorption spectrum of the measurement target gas X is measured and the concentration is calculated from the measured area. In addition, the calculation controller 22 drives and controls the display section D and displays a plurality of the measurement results of the measurement target gas X (the components which are included in the measurement target gas X and the concentrations thereof, the transmittance of the laser light L, the temperature of the measurement target gas X, and the like) on the display section D. It is possible to form the calculation controller 22 of a CPU, FPGA, or the like.

The memory 23 is a non-volatile memory such as a flash read-only memory (ROM) or an electrically erasable and programmable ROM (EEPROM) and stores various types of data. For example, the memory 23 stores data (for example, data which indicates the absorption spectrums of ethylene, methane, and ethane in a certain wavelength region) which is used when the calculation controller 22 calculates the components which are included in the measurement target gas X and the concentrations thereof.

The output section 24 is connected to the input section 12 of the light emitter 10 via the cable CB and outputs a transmittance signal which indicates the transmittance of the laser light L which is obtained in the calculation controller 22. The transmittance signal is a digital signal and is transmitted to the light emitter 10 via the cable CB without being influenced by noise.

Next, a brief description will be given of an operation of the gas concentration analyzer 1 with the configuration described above during the measurement. When the measurement is started, firstly, the light emitting module 11 is controlled by the controller 13 and the emission of the laser light L is started. Then, the laser light L with a line width (a spectrum line width) of approximately 0.2 nm which has a wavelength within the wavelength regions R1 to R4 shown in Fig. 5A to Fig. 7 is emitted from the light emitting module 11.

The laser light L which is emitted from the light emitting module 11 is guided to the pipeline P via the inside of the branch pipeline B1 (refer to Fig. 8). In the laser light L which is guided to the pipeline P, the laser light L which passes through the measurement target gas X which flows in the pipeline P is guided to the light receiver 20 via the branch pipeline B2 (refer to Fig. 8) and received in the light receiving module 21. When the laser light L is received in the light receiving module 21, a light receiving signal is output to the calculation controller 22.

In addition, when the emission of the laser light L is started, the light emitting module 11 is controlled by the controller 13 such that the wavelength of the laser light L is tuned (so as to sweep a predetermined wavelength range within the wavelength regions R1 to R4 described above). Here, even when the wavelength of the laser light L is tuned, the line width (the spectrum line width) of the laser light L is maintained to be approximately 0.2 nm. After the laser light L of which the wavelength is tuned is also guided to the pipeline P and passes through the measurement target gas X which flows in the pipeline P, the laser light L is guided to the light receiver 20 and received in the light receiving module 21. When the laser light L is received in the light receiving module 21, a light receiving signal is output to the calculation controller 22.

Every time the wavelength of the laser light L is tuned, the same operation as the operation described above is performed and, due to this, an absorption spectrum which indicates changes in the strength of the light receiving signal according to the wavelength is obtained. When the absorption spectrum is obtained, a process of measuring the concentration of the components which are included in the measurement target gas X is performed in the calculation controller 22 using the 2f method or the spectrum area method described above. In detail, a process of simultaneously measuring the concentration of trace amounts of methane and ethane which are included in ethylene as the measurement target gas X is performed. When the process is completed, the concentration of trace amounts of methane and ethane which are included in ethylene is displayed on the display section D.

As described above, in the present embodiment, the light in the wavelength region R1 (3.42 µm to 3.71 µm) is irradiated with respect to the measurement target gas X (ethylene in which trace amounts of ethane and methane are included) and the concentration of the measurement target gas X is measured based on the absorbance of the laser light L which passes through the measurement target gas X. In the wavelength region R1, since the absorbance of ethylene is small and the absorbance of methane and ethane is high, it is possible to measure trace amounts of methane and ethane which are included in ethylene with high precision.

Here, in the wavelength region R2 (3.42 µm to 3.50 µm) which is included in the wavelength region R1 described above, since the absorbance of ethane is great and the shape of the absorption spectrum of methane is a characteristic shape, it is possible to easily measure methane and ethane when using the light in the wavelength region R2. In addition, in the wavelength region R3 (3.439 µm to 3.442 µm) or the wavelength region R4 (3.451 µm to 3.455 µm) which are included in the wavelength region R2 described above, not only is the absorbance of ethane greater than the absorbance of ethylene, but the absorbance of methane is also great and the absorbance of methane is approximately the same as the absorbance of ethylene (or the absorbance of methane is greater than the absorbance of ethylene). In addition, the shape of the absorption spectrum of methane and ethane is a characteristic shape. For this reason, when using the light in the wavelength region R3 or the wavelength region R4, it is possible to measure trace amounts of methane and ethane which are included in ethylene with high precision.

In addition, in the present embodiment, by using the light in the wavelength region R1 described above, it is possible to use the light source and light receiving elements for measuring methane and the light source and light receiving elements for measuring ethane together. In other words, it is not necessary to separately provide the light source and light receiving elements for measuring methane and the light source and light receiving elements for measuring ethane. For this reason, it is possible to measure trace amounts of methane and ethane which are included in ethylene without increasing the cost.

Description was given above of the gas concentration analyzer according to one embodiment of the present invention; however, it is possible to freely change the present invention within the range of the present invention without being limited to the embodiment described above. For example, in the embodiment described above, examples of the gas concentration analyzer 1 include a laser gas analyzer; however, the gas concentration analyzer of the present invention is not limited to a laser gas analyzer. Apart from a laser gas analyzer, the gas concentration analyzer of the present invention may be, for example, an arbitrary gas concentration analyzer such as a gas concentration analyzer which uses Fourier transform infrared spectrophotometer (FT-IR), a gas concentration analyzer which uses a spectroscope which uses a filter, and a gas concentration analyzer which uses a spectroscope which uses grating.

As used herein, the following directional terms "forward, rearward, above, downward, right, left, vertical, horizontal, below, transverse, row and column" as well as any other similar directional terms refer to those directions of an apparatus equipped with the present invention. Accordingly, these terms, as utilized to describe the present invention should be interpreted relative to an apparatus equipped with the present invention.

The term "configured" is used to describe a component, unit or part of a device includes hardware and/or software that is constructed and/or programmed to carry out the desired function.

Moreover, terms that are expressed as "means-plus function" in the claims should include any structure that can be utilized to carry out the function of that part of the present invention.

The term "unit" is used to describe a component, unit or part of a hardware and/or software that is constructed and/or programmed to carry out the desired function. Typical examples of the hardware may include, but are not limited to, a device and a circuit.

While preferred embodiments of the present invention have been described and illustrated above, it should be understood that these are examples of the present invention and are not to be considered as limiting. Additions, omissions, substitutions, and other modifications can be made without departing from the scope of the present invention. Accordingly, the present invention is not to be considered as being limited by the foregoing description, and is only limited by the scope of the claims.

## Claims

1. A gas concentration analyzer comprising:
a light emitting module configured to emit a laser light with which an ethylene gas that is a measurement target gas is irradiated;
a light receiving module configured to receive the laser light that has passed through the measurement target gas to output a light receiving signal; and
a calculation controller configured to calculate an absorption spectrum of the laser light using the light receiving signal, and to calculate amounts of methane and amounts of ethane in the ethylene gas that is the measurement target gas based on the absorption spectrum,
wherein the laser light with which the measurement target gas is irradiated is included in a wavelength region with a wavelength of 3.42 µm or more and 3.71 µm or less.

2. The gas concentration analyzer according to claim 1, wherein the laser light with which the measurement target gas is irradiated is included in a wavelength region with a wavelength of 3.42 µm or more and 3.50 µm or less.

3. The gas concentration analyzer according to claim 2, wherein the laser light with which the measurement target gas is irradiated is included in a wavelength region with a wavelength of 3.439 µm or more and 3.442 µm or less or 3.451 µm or more and 3.455 µm or less.

4. The gas concentration analyzer according to claim 1 (any one of claims 1 to 3), wherein the laser light with which the measurement target gas is irradiated has a spectrum line width which is equivalent to or less than a spectrum line width of an absorption peak of methane or ethane.

5. The gas concentration analyzer according to claim 4, comprising:
a tunable wavelength light source configured to be capable of changing the wavelength of the laser light with which the measurement target gas is irradiated within the wavelength region.

6. The gas concentration analyzer according to claim 5, comprising:
only one tunable wavelength light source,
wherein the methane and the ethane which are included in the ethylene gas that is the measurement target gas can be calculated simultaneously using the laser light emitted from the tunable wavelength light source.

7. The gas concentration analyzer according to claim 6, wherein the tunable wavelength light source is a quantum cascade laser (QCL) or an interband cascade laser (ICL).

8. A gas concentration analysis method comprising:
emitting a laser light with which an ethylene gas that is a measurement target gas is irradiated;
receiving the laser light that has passed through the measurement target gas to output a light receiving signal;
calculating an absorption spectrum of the laser light using the light receiving signal; and
calculating amounts of methane and amounts of ethane in the ethylene gas that is the measurement target gas based on the absorption spectrum,
wherein the laser light with which the measurement target gas is irradiated is included in a wavelength region with a wavelength of 3.42 µm or more and 3.71 µm or less.

9. The gas concentration analysis method according to claim 8, wherein the laser light with which the measurement target gas is irradiated is included in a wavelength region with a wavelength of 3.42 µm or more and 3.50 µm or less.

10. The gas concentration analysis method according to claim 9, wherein the laser light with which the measurement target gas is irradiated is included in a wavelength region with a wavelength of 3.439 µm or more and 3.442 µm or less or 3.451 µm or more and 3.455 µm or less.

11. The gas concentration analysis method according to claim 8 (any one of claims 8 to 10), wherein the laser light with which the measurement target gas is irradiated has a spectrum line width which is equivalent to or less than a spectrum line width of an absorption peak of methane or ethane.

12. The gas concentration analysis method according to claim 11, wherein the wavelength of the light with which the measurement target gas is irradiated can be tuned within the wavelength region by a tunable wavelength light source.

13. The gas concentration analysis method according to claim 12, wherein the methane and the ethane, which are included in the ethylene, can be calculated simultaneously using the laser light which is emitted from the tunable wavelength light source.

14. The gas concentration analysis method according to claim 13, wherein the tunable wavelength light source is a quantum cascade laser (QCL) or an interband cascade laser (ICL).
